# EUROPEAN PATENT APPLICATION

(11) **EP 4 817 565 A1**
(43) Date of publication of application: **07.10.2026**
(21) Application number: 25174736.6
(22) Date of filing: 07.05.2025
(51) Int. Cl.: A61F 2/66, A61F 2/70, A61F 2/76, A61H 3/00

(54) **UNIFIED CONTROLLER FOR POWERED ANKLE ASSISTIVE DEVICES**

(30) Priority: 04.04.2025 US 202519170523
(71) Applicant: University of Utah Research Foundation, Salt Lake City, UT 84112 (US)
(72) Inventor: LENZI, Tommaso, Salt Lake City, 84112 (US); PRUYN, Kai, Salt Lake City, 84112 (US)
(74) Representative: Forresters IP LLP

(57) **Abstract**

A powered ankle support device, for example, an ankle prosthesis or ankle exoskeleton, comprises an ankle joint assembly coupled between an upper support member, such as the shank, and a lower support member, such as a prosthetic foot or foot support member. An actuator is associated with the ankle joint assembly to move the lower support member with respect to the upper support member. Angle sensors sense an ankle angle of the ankle joint assembly, a shank angle of the upper support member, a knee angle of a knee associated with the ankle joint assembly, and a thigh angle of a thigh associated with the ankle joint assembly. A controller is associated with the ankle joint assembly and receives angle data from the angle sensors comprising the ankle angle, the shank angle, the knee angle and the thigh angle. The controller determines a torque to be applied by the actuator based on the angle data. The controller generates a control signal to the actuator to enable the actuator to apply the torque between the ankle joint assembly and the lower support member.

## Description

### CROSS REFERENCE TO RELATED APPLICATIONS

This application claims priority to U.S. Patent Application No. 19/170,523, filed April 4, 2025.

### STATEMENT REGARDING FEDERALLY SPONSORED

### RESEARCH OR DEVELOPMENT

This invention was made with government support under R01 HD098154 awarded by the National Institutes of Health, and 2046287 awarded by the National Science Foundation. The government has certain rights in the invention.

### NAMES OF THE PARTIES TO A JOINT RESEARCH AGREEMENT

Not applicable.

### INCORPORATION BY REFERENCE STATEMENT

Not applicable.

### BACKGROUND

The quality of life and ambulation ability of over 1 million people is affected by lower limb amputation in the United States. Currently available passive prostheses use springs and dampers to provide torque at the joint level. These prostheses can be reliable and lightweight; however, they may not inject net positive energy into the gait cycle. This makes many ambulation activities difficult to perform and consequently contributes to compensatory ambulation movements which can negatively affect long term health. To address this issue, researchers are currently developing fully powered prostheses that can inject net positive energy into the gait cycle. Some powered prostheses, however, rely on finite state machine control methods that depend upon determining a state or phase of the gait cycle. Such gait cycle prostheses can experience difficulties with uneven or variable terrain.

Amputation can affect the quality of life of the individual. Amputation can also affect mobility of the individual. For example, the average able-bodied individual walks 5100 steps per day. Whereas the average amputee walks only 3000 steps per day. Amputees with a transtibial or below knee amputation spend more energy than able bodied individuals and still walk slower than able-bodied individuals. Below knee amputations also make it more difficult to do other activities besides walk, such as climb stairs and ramps, and stand up from a sitting position.

Many types of ankle prostheses have been developed attempting to restore mobility post amputation. Today the most commercially available are passive prosthetics. The most fundamental version of a passive prosthesis is a simple stiffness element often made up of a carbon fiber spring. These carbon fiber foot keels can be extremely lightweight. However, they can also have a fixed equilibrium position that is tuned for more common level ground ambulation activities, such as walking and standing. This poses a problem for ambulation activities like stairs, ramps, and sit-to-stand transitions, because they require greater ranges of motion. Using a stiffness element prosthesis on inclined terrain can create increased instability due to a lack of range of motion. A newer category of passive prosthesis employs a joint with passive elements like springs and dampers. Microprocessor ankles use a microprocessor to tune the dampers according to onboard sensors. These types of devices, however, neglect that during the push-off phase of ambulation, the ankle provides net positive energy. They can also be incapable of actively positioning the joint during the swing. For example, positioning the ankle during swing in stair ascent for toe clearance reduces the likelihood of tripping. Positioning in-swing can also aid in adapting to inclined terrain. Because passive devices are energetically passive, they may not actively control these movements. This can often lead to the employment of unnatural compensatory movements to compensate for the lack of net positive energy. For example, on stairs, amputees often cannot ascend step-over-step like able bodied individuals. Rather, they ascend one step at a time, taking each step with their intact limb first. To clear the steps, the amputee also may extend and circumduct in an unnatural way.

Fully powered prosthetics can provide net positive energy where passive prosthetics cannot. These fully powered prosthetics can employ, batteries, motors, and clever transmission systems to provide power at the ankle joint. As the name implies, these devices may rely on their active components to power all ambulation activities. These devices may actively provide push-off and actively control swing. This ability may lead to an amputee being able to perform stairs in a step-over-step fashion. However, because some ambulation activities require high torque, and some ambulation activities require high speed, powering all ambulation activities often leads to demanding motor requirements. The added components, like heavy motors, large batteries, and bulky transmission components may lead to heavier fully-powered prosthesis. Increasing weight may be a problem because increasing prosthetic weight may lead to increased metabolic cost.

Notably, many fully intact individuals experience a variety of dysfunction in the lower limbs. For example, stroke commonly results in hemiparesis (~80% of stroke survivors) which results in severe muscle weakness in one side of the body. Individuals with hemiparesis have many of the same impairments as amputees, including a higher energy cost of walking, increased fall risk, and asymmetric gait patterns. The nonparetic side must compensate for the weakness of the paretic side, and individuals post-stroke commonly use compensatory movements. Ankle exoskeletons have been used to assist such hemiparetic gait in the past. However, available ankle exoskeletons have similar limitations to those discussed for prostheses. Further, current exoskeleton control methods involve instrumented foot plates which are used to tell whether the foot is in contact with the ground. Ground contact information is then used to determine if the user is in a swing or stance state/phase of gait. Ground force sensors and instrumented foot plates are expensive and tend to be unreliable. Finite state machine control methods depend upon determining a state or phase of the gait cycle. Such gait cycle exoskeletons and prostheses can experience difficulties with uneven or variable terrain. As such, solutions continue to be evaluated and sought to improve gait performance.

### SUMMARY

This invention relates to an active powered ankle assistive device, such as an ankle prosthesis or an ankle exoskeleton, with a unified controller to provide plantarflexion and dorsiflexion assistance throughout the gait cycle and adapt to variable terrains. An ankle joint assembly is configured to be coupled between an upper support member and a lower support member. An actuator is associated with the ankle joint assembly and configured to move the lower support member with respect to the upper support member. One or more angle sensors are configured to sense an ankle angle of the ankle joint assembly, a shank angle of the upper support member, a knee angle of a knee associated with the ankle joint assembly, and a thigh angle of a thigh associated with the ankle joint assembly. A controller is associated with the ankle joint assembly and is configured to receive angle data from the one or more angle sensors comprising the ankle angle, the shank angle, the knee angle and the thigh angle. The controller is configured to determine a torque to be applied by the actuator based on the angle data. The controller is configured to generate a control signal to the actuator to enable the actuator to apply the torque between the ankle joint assembly and the lower support member.

There has thus been outlined, rather broadly, the more important features of the invention so that the detailed description thereof that follows may be better understood, and so that the present contribution to the art may be better appreciated. Other features of the present invention will become clearer from the following detailed description of the invention, taken with the accompanying drawings and claims, or may be learned by the practice of the invention.

### BRIEF DESCRIPTION OF THE DRAWINGS

FIG. 1 is a schematic diagram of a side view of an example powered ankle assistive device, namely an ankle prosthetic, with a controller, according to some embodiments.
FIG. 2 is a schematic diagram of example torque applied through a gait cycle by the ankle prosthetic of FIG. 1, according to some embodiments.
FIG. 3 is a schematic plot of example ankle torque (T_{A}) versus ankle angle (θ_{A}) for stiffness values (K1 and K2) of the ankle prosthetic of FIG. 1, according to some embodiments.
FIG. 4 is a schematic plot of example values of a variable (α) versus knee angle (θ_{K}) of the ankle prosthetic of FIG. 1, according to some embodiments.
FIG. 5 is a schematic plot of example values of a variable (β) versus thigh angle (θₜₕ) of the ankle prosthetic of FIG. 1, according to some embodiments.
FIG. 6 is a schematic plot of example equilibrium angles (θ_{eq}) versus knee angles (θ_{K}) for a synergistic gain value (K_{syn}) of the ankle prosthetic of FIG. 1, according to some embodiments.
FIG. 7 is a schematic plot of example thigh angle gain values (Kₜₕ) versus thigh angle (θₜₕ) of the ankle prosthetic of FIG. 1, according to some embodiments.
FIG. 8 is a schematic diagram of a side view of an example powered ankle assistive device, namely an ankle exoskeleton, with a controller, according to some embodiments.

These drawings are provided to illustrate various aspects of the invention and are not intended to be limiting of the scope in terms of dimensions, materials, configurations, arrangements or proportions unless otherwise limited by the claims.

### DETAILED DESCRIPTION

While these exemplary embodiments are described in sufficient detail to enable those skilled in the art to practice the invention, it should be understood that other embodiments may be realized and that various changes to the invention may be made without departing from the spirit and scope of the present invention. Thus, the following more detailed description of the embodiments of the present invention is not intended to limit the scope of the invention, as claimed, but is presented for purposes of illustration only and not limitation to describe the features and characteristics of the present invention, to set forth the best mode of operation of the invention, and to sufficiently enable one skilled in the art to practice the invention. Accordingly, the scope of the present invention is to be defined solely by the appended claims.

### Definitions

In describing and claiming the present invention, the following terminology will be used.

The singular forms "a," "an," and "the" include plural referents unless the context clearly dictates otherwise. Thus, for example, reference to "a motor" includes reference to one or more of such materials and reference to "engaging" refers to one or more of such actions.

As used herein with respect to an identified property or circumstance, "substantially" refers to a degree of deviation that is sufficiently small so as to not measurably detract from the identified property or circumstance. The exact degree of deviation allowable may in some cases depend on the specific context.

As used herein, "adjacent" refers to the proximity of two structures or elements. Particularly, elements that are identified as being "adjacent" may be either abutting or connected. Such elements may also be near or close to each other without necessarily contacting each other. The exact degree of proximity may in some cases depend on the specific context.

As used herein, "assistive device" refers to a biomechanical device which is connected to a human in order to augment or replace limb movement. In the case of augmentation, the device is configured as an exoskeleton which is secured to an existing limb to support and augment movement of an existing joint or limb. In the case of replacement, the device is configured as a prosthesis which is secured to an individual at a point of amputation. Non-limiting examples of joints which can be augmented or replaced include ankles, knees, hips, and the like.

As used herein, the term "about" is used to provide flexibility and imprecision associated with a given term, metric, or value. The degree of flexibility for a particular variable can be readily determined by one skilled in the art. However, unless otherwise enunciated, the term "about" generally connotes flexibility of less than 2%, and most often less than 1%, and in some cases less than 0.01%.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a *de facto* equivalent of any other member of the same list solely based on their presentation in a common group without indications to the contrary.

As used herein, the term "at least one of" is intended to be synonymous with "one or more of." For example, "at least one of A, B and C" explicitly includes only A, only B, only C, or combinations of each.

Numerical data may be presented herein in a range format. It is to be understood that such range format is used merely for convenience and brevity and should be interpreted flexibly to include not only the numerical values explicitly recited as the limits of the range, but also to include all the individual numerical values or sub-ranges encompassed within that range as if each numerical value and sub-range is explicitly recited. For example, a numerical range of about 1 to about 4.5 should be interpreted to include not only the explicitly recited limits of 1 to about 4.5, but also to include individual numerals such as 2, 3, 4, and sub-ranges such as 1 to 3, 2 to 4, etc. The same principle applies to ranges reciting only one numerical value, such as "less than about 4.5," which should be interpreted to include all of the above-recited values and ranges. Further, such an interpretation should apply regardless of the breadth of the range or the characteristic being described.

Any steps recited in any method or process claims may be executed in any order and are not limited to the order presented in the claims. Means-plus-function or step-plus-function limitations will only be employed where for a specific claim limitation all of the following conditions are present in that limitation: a) "means for" or "step for" is expressly recited; and b) a corresponding function is expressly recited. The structure, material or acts that support the means-plus function are expressly recited in the description herein. Accordingly, the scope of the invention should be determined solely by the appended claims and their legal equivalents, rather than by the descriptions and examples given herein.

### Example Embodiments

A technology is described for a unified controller for an ankle joint of an ankle prosthesis or an ankle exoskeleton that can provide plantarflexion and dorsiflexion assistance throughout a gait cycle and that can adapt to variable terrains, such as ramps and stairs. The controller can have two parts including a first part that provides positive dorsiflexion torque associated with swing during the gait cycle, and a second part that can provide assistance associated with stance during gait cycle. Specifically, the second part of the controller can provide dorsiflexion assistance associated with early to mid-stance and negative plantarflexion torque associated with a mid-to-late stance or a propulsive phase of the gait cycle. In order to determine which part of the controller is actively on and providing assistance, a series of thresholds based on the knee angle *θ_{K}* and thigh angle *θₜₕ* can be implemented. The angles of the ankle *θ_{A}*, shank *θₛₕ,* knee *θ_{K},* and thigh *θₜₕ* can be used to determine a magnitude and a timing of the assistance.

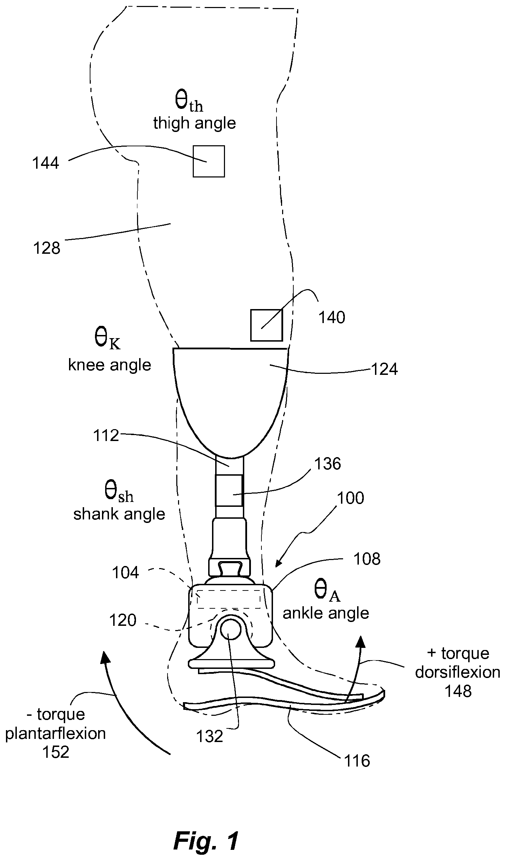

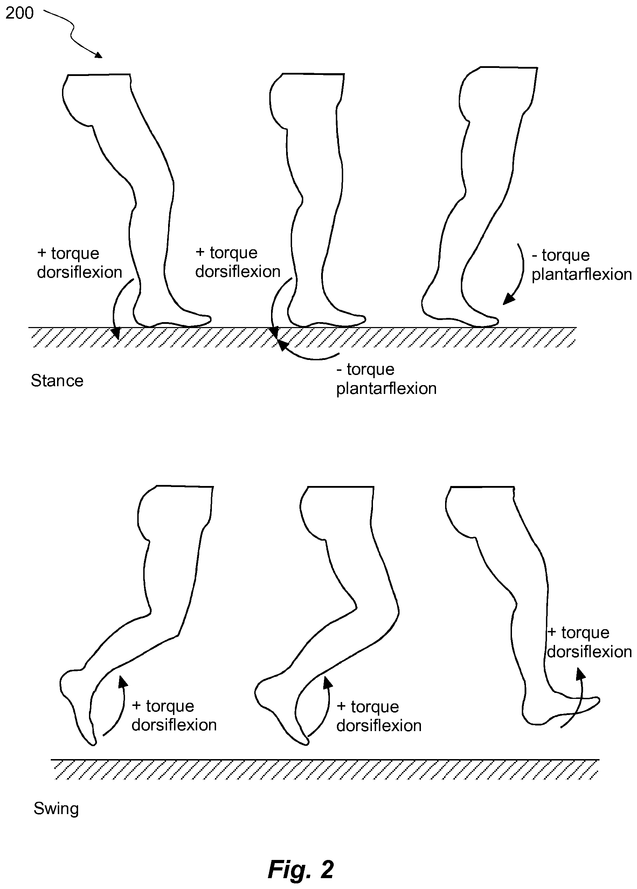

In one specific example, FIG. 1 illustrates a powered ankle assistive device, and namely an ankle prosthesis 100, with a unified controller 104 in one example of the invention. FIG. 2 illustrates operation of the unified controller 104 through a gait cycle 200. The ankle prosthesis 100 can comprise an ankle joint assembly 108 configured to be coupled between an upper support member, such as a shank 112, and a lower support member, such as a prosthetic foot member 116. The ankle prosthesis 100 can be powered and/or active. Thus, the ankle prosthesis 100 can have an actuator 120 associated with the ankle joint assembly 108 and configured to move the prosthetic foot member 116 with respect to the shank 112. The actuator 120 can comprise one or more electric, hydraulic and/or pneumatic linear and/or rotary motors coupled to and between the ankle joint assembly 108 and the prosthetic foot member 116. The prosthetic foot member 116 can be coupled to the ankle joint assembly 108 at a pivot axle. The prosthetic foot member 116 can be passive and can comprise one or more composite leaf springs.

The ankle prosthesis 100 can comprise one or more angle sensors configured to sense an ankle angle θ_{A} of the ankle joint assembly 108, a shank angle θₛₕ of the shank 112, a knee angle θ_{K} of a knee 124 associated with the ankle joint assembly 108, and a thigh angle θₜₕ of a thigh 128 associated with the ankle joint assembly 108. The one or more angle sensors can comprise an ankle angle sensor 132 to measure the ankle angle θ_{A}, a shank angle sensor 136 to measure the shank angle θₛₕ, a knee angle sensor 140 to measure the knee angle θ_{K}, and a thigh angle sensor 144 to measure the thigh angle θₜₕ. The ankle angle sensor 132 can be located at the ankle joint assembly 108, the shank angle sensor 136 can be located at the shank 112, the knee angle sensor 140 can be located at the knee 124 of an amputee, and the thigh angle sensor 144 can be located at the thigh 128 of the amputee. The angle sensor for a particular angle can comprise one or more angle sensors. The angle sensor can measure an angle between proximate limbs or an angle with respect to a reference, such as vertical. The angle sensor can be one or more of a joint encoder, a digital goniometer, or an inertial measurement unit (IMU). The angle sensors can be operatively or communicatively coupled to the controller 104, such as electrically or wirelessly.

The controller 104 can be associated with the ankle joint assembly 108. In one aspect, the controller 104 can be carried by ankle joint assembly 108. The ankle joint assembly 108 can also carry a power source, such as a rechargeable battery to power the actuator 120 and the controller 104.

The controller 104 can comprise one or more processors coupled to a memory. The controller 104 can be configured to receive angle data from the one or more angle sensors. The angle data can comprise the ankle angle θ_{A}, the shank angle θₛₕ, the knee angle θ_{K} and the thigh angle θₜₕ. The controller 104 can be configured to determine a torque to be applied by the actuator 120 based on the angle data, as discussed in greater detail below. The controller 104 can be configured to generate a control signal to the actuator 120 to enable the actuator to apply the torque between the ankle joint assembly 108 and the prosthetic foot member 116.

The controller 104 can be further configured to determine a positive (+) dorsiflexion torque 148 based on the angle data associated with swing during gait. The control signal can enable the actuator 120 to apply the positive dorsiflexion torque 148. The controller 104 can be further configured to determine a positive dorsiflexion torque 148 or a negative (-) plantarflexion torque 152 based on the angle data associated with a stance or propulsive phase of gait. The control signal can enable the actuator to apply the positive dorsiflexion torque 148 or the negative plantarflexion torque 152.

The controller 104 can be further configured to determine a magnitude and a timing of the torque applied by the actuator 120 based on only the angle data. The controller 104 can be further configured to generate the control signal to enable the actuator 120 to continuously apply torque between the ankle joint assembly 108 and the prosthetic foot member 116.

The controller 104 can be further configured to determine an ankle velocity *θ̇_{A}* based on a change in the ankle angle θ_{A} over time. Thus, the ankle velocity *θ̇_{A}* can be determined based on the ankle angle θ_{A}.

A desired ankle torque ${T}_{A}^{Des}$ can be determined by a stiffness coefficient K_{S} and a damping coefficient B that can be tuned. The stiffness coefficient K_{S} can be applied to a difference in an actual ankle angle θ_{A} and a desired ankle equilibrium angle θ_{eq}. The damping coefficient B can be multiplied by an ankle velocity *θ̇_{A}.* Thus, the desired ankle torque ${T}_{A}^{Des}$ can be determined by: ${T}_{A}^{Des} = {K}_{S} \left({θ}_{A}-{θ}_{eq}\right) + B {\dot{θ}}_{A}$ where:
T_{A} is the torque applied;
K_{S} is the stiffness coefficient;
θ_{A} is the ankle angle;
θ_{eq} is the ankle equilibrium angle;
B is the damping coefficient; and
*θ̇*_{A} is the ankle velocity.

In one aspect, the controller 104 can be further configured to determine the torque T_{A} to be applied by the actuator 120 based on a stiffness coefficient K_{S} applied to a difference between the ankle angle θ_{A} and an ankle equilibrium angle θ_{eq}; and a damping coefficient B applied to an ankle velocity *θ̇*_{A}. As discussed above, the ankle velocity *θ̇*_{A} can be determined by the controller 104 based on the change in the ankle angle θ_{A} over time.

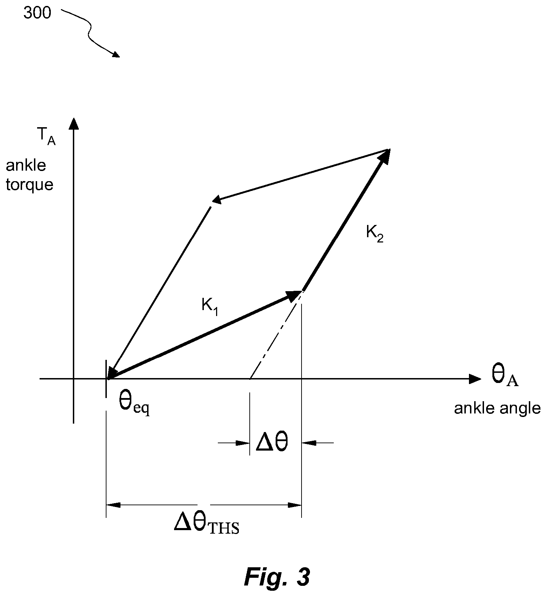

FIG. 3 illustrates the stiffness coefficient K_{S} on a plot 300 of ankle torque T_{A} versus ankle angle θ_{A}. The stiffness coefficient K_{S} can comprise two stiffness values, K1 and K2, and can be decreased as the actual ankle angle θ_{A} approaches the desired equilibrium angle θ_{eq}. The two stiffness values, K1 and *K2,* and the parameters Δ*θ* and Δ*θths,* can be tuned. The stiffness coefficient can be implemented for both plantarflexion, e.g. K_{PF1} and K_{PF2}, and dorsiflexion, e.g. K_{DF1} and K_{DF2}. Thus, the different stiffness values can be tuned based on whether the ankle is plantarflexed or dorsiflexed.

In another aspect, the stiffness coefficient K_{S} can decrease as the ankle angle approaches the ankle equilibrium angle θ_{A}. In another aspect, the stiffness coefficient K_{S} can comprise at least two different stiffness coefficients K1 and K2 corresponding to different ranges of the ankle angle θ_{A} associated with different stiffness values, and comprising a first stiffness coefficient K1 corresponding to a first range of ankle angles and a second stiffness coefficient K2 corresponding to a second range of ankle angles. In another aspect, the second stiffness coefficient K2 can be greater than the first stiffness coefficient K1, and the ankle angles θ_{A} in the second range of ankle angles can be greater than the ankle angles θ_{A} in the first range of ankle angles. In another aspect, the stiffness coefficient for each of plantarflexion and dorsiflexion comprises at least two different stiffness coefficients corresponding to different ranges of the ankle angle.

The damping coefficient B depends on whether the ankle velocity *θ̇_{A}* is positive (dorsiflexing) *B_{DF}* or negative (plantarflexing) *B_{PF}.* Both damping coefficient values, *B_{DF}* and *B_{PF},* can be tuned. The damping coefficient B can be determined by: If ${\dot{θ}}_{A} < 0 ,$ then $B = {B}_{PF} ,$ else $B = {B}_{DF} .$

In one aspect, the damping coefficient B can comprise a dorsiflexion damping coefficient *B_{DF}* corresponding to a positive velocity of the ankle associated with dorsiflexing, and a different plantarflexion damping coefficient *B_{PF}* corresponding to a negative velocity of the ankle associated with plantarflexing.

The desired equilibrium angle θ_{eq} can be based on a sum of two equilibrium angles, θ_{eq1} and θ_{eq2}, that turn on and off based on the knee angle θ_{K} and the thigh angle θₜₕ. The knee and thigh angles, θ_{K} and θₜₕ, can indicate whether the leg is in stance or swing during gait. During stance, the knee is straight, and the thigh starts in front of the body (of an amputee with the ankle prosthesis) and moves behind the body. During swing, the knee is bent, and the thigh is in front of the body. A first equilibrium angle θ_{eq1} can be associated with dorsiflexion assistance during early-to-mid stance and plantarflexion assistance during mid-to-late stance, and turns off during swing. A second equilibrium angle θ_{eq2} can be associated with dorsiflexion assistance during swing and turns off during stance. The equilibrium angle θ_{eq} can be determined by: ${θ}_{eq} = {θ}_{eq 1} + {θ}_{eq 2}$ where:
θ_{eq} is the equilibrium angle;
θ_{eq1} is a first equilibrium angle corresponding to application of positive dorsiflexion torque and negative plantarflexion torque and associated with an early-to-mid stance and a mid-to-late stance during gait, respectively; and
θ_{eq2} is a second equilibrium angle corresponding to application of positive dorsiflexion torque and associated with swing.

In one aspect, the equilibrium angle θ_{eq} can be based on the knee angle θ_{K} and the thigh angle θₜₕ. In another aspect, the equilibrium angle θ_{eq} can be comprised of two equilibrium angles θ_{eq1} and θ_{eq2}. The first equilibrium angle θ_{eq1} can correspond to application of positive dorsiflexion torque and negative plantarflexion torque and can be associated with an early-to-mid stance and a mid-to-late stance, respectively. The second equilibrium angle θ_{eq2} can correspond to application of positive dorsiflexion torque and can be associated with a swing of gait.

The first half of the desired equilibrium angle equation, θ_{eq1}, corresponds to both plantarflexion and dorsiflexion assistance. The first equilibrium angle θ_{eq1} can be equal to a threshold gain value Kₜₕₛ₁ multiplied by a difference in the ankle angle θ_{A} and the shank angle θₛₕ. The first equilibrium angle θ_{eq1} can be determined by: ${θ}_{eq 1} = {K}_{ths 1} \left({θ}_{A}-{θ}_{sh}\right) ;$ where:
Kₜₕₛ₁ is a threshold gain value;
θ_{A} is the ankle angle; and
θₛₕ is the shank angle.

When the threshold gain value Kₜₕₛ₁ is equal to 0, the first equilibrium angle θ_{eq1} is equal to 0. The threshold gain value Kₜₕₛ₁ can be based on the knee and thigh angles, θ_{K} and θₜₕ, that set values for variables *α* and *β*. If both variables *α* and *β* are equal to 1, this half of the equilibrium angle equation, θ_{eq1}, turns off. The threshold gain value Kₜₕₛ₁ can be determined by: ${K}_{ths 1} = 1 - αβ ;$ where:
α has a value increasing from 0 to 1 as the knee angle θ_{K} increases between two threshold values; and
β has a value increasing from 0 to 1 as the thigh angle θₜₕ increases between two threshold values.

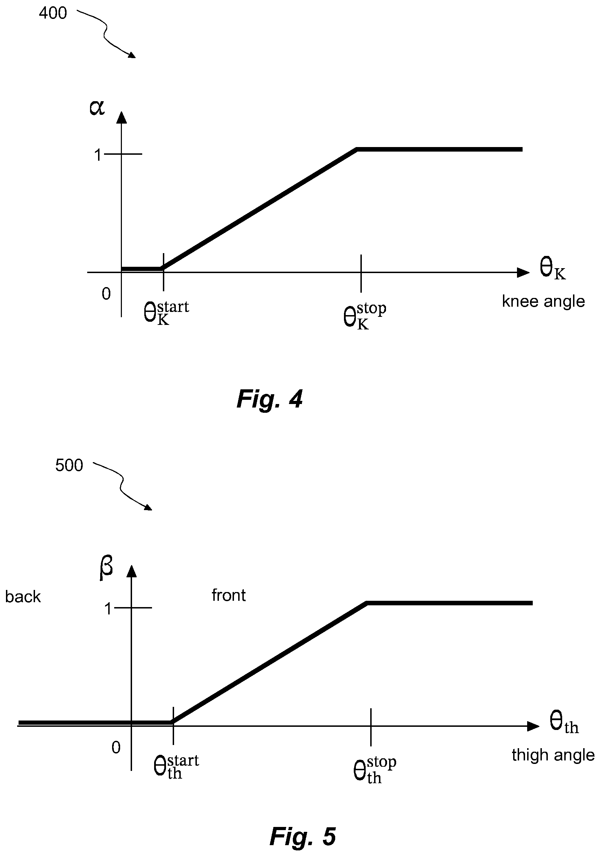

FIG. 4 illustrates plot 400 of example values of the variable α versus the knee angle θ_{K}. The knee angle θ_{K} can determine the value of α, increasing from 0 to 1 as the knee angle θ_{K} increases between two threshold values, ${θ}_{K}^{start}$ and ${θ}_{K}^{stop}$*,* that can be tuned.

FIG. 5 illustrates plot 500 of example values of the variable β versus the thigh angle θₜₕ. The thigh angle θₜₕ can determine the value of β, increasing from 0 to 1 as the thigh angle θₜₕ increases between two threshold values, ${θ}_{th}^{start}$ and ${θ}_{th}^{start}$*,* that can be tuned. The thigh angle θₜₕ is positive when the thigh is in front of the body and negative when the thigh is behind the body.

Thus, when both variables α and β are 1, the threshold gain value Kₜₕₛ₁ is equal to 0. This turns off the first half of the desired equilibrium angle equation, θ_{eq1}, during swing, when the thigh is in front of the body and the knee is bent. During stance, from heel strike to toe-off, the threshold gain value Kₜₕₛ₁ is equal to 1, and the desired equilibrium angle equation, θ_{eq1}, is equal to the difference between the ankle and shank angles, θ_{A} and θₛₕ. This difference is positive during early stance, so the first equilibrium angle θ_{eq1} contributes to dorsiflexion assistance. This difference becomes negative during mid-to-late stance, so the first equilibrium angle θ_{eq1} begins contributing to plantarflexion assistance. During push-off at the end of the stance, this difference is most negative, and the first equilibrium angle θ_{eq1} provides a maximum amount of plantarflexion assistance. Basing the stance assistance on the difference between the ankle and shank angles, θ_{A} and θₛₕ, also allows the ankle exoskeleton assistance to adapt to different inclines and variable terrains that change the ankle angle θ_{A} relative to the shank angle θₛₕ during stance.

In one aspect, the first equilibrium angle θ_{eq1} can equal a threshold gain value Kₜₕₛ₁ multiplied by a difference between the ankle angle and the shank angle, θ_{A} and θₛₕ. The threshold gain value Kₜₕₛ₁ can equal 1 less a product of knee angle factor α increasing from 0 to 1 as the knee angle θ_{K} increases between two threshold values and thigh angle factor β increasing from 0 to 1 as the thigh angle θₜₕ increases between two threshold values.

The second half of the desired equilibrium angle equation, θ_{eq2}, provides only dorsiflexion assistance during swing. The second equilibrium angle θ_{eq2} can be calculated from a synergistic gain value K_{syn} based on the knee angle θ_{K} and the current equilibrium angle θ_{eq}, as well as a gain value Kₜₕ based on the thigh angle θₜₕ. The second equilibrium angle θ_{eq2} can be determined by: ${θ}_{eq 2} = {K}_{syn} {K}_{th} ;$ where:
K_{syn} is a slope of a line between two knee angle thresholds and two equilibrium angle thresholds, and where K_{syn} has a value of 0 when the knee angle is straight associated with stance during gait and where K_{syn} increases as the knee angle bends faster during swing during gait; and
Kₜₕ has a value increasing from 0 to 1 as the thigh angle changes with the thigh in front of the body and has a value of 0 when the thigh is behind the body.

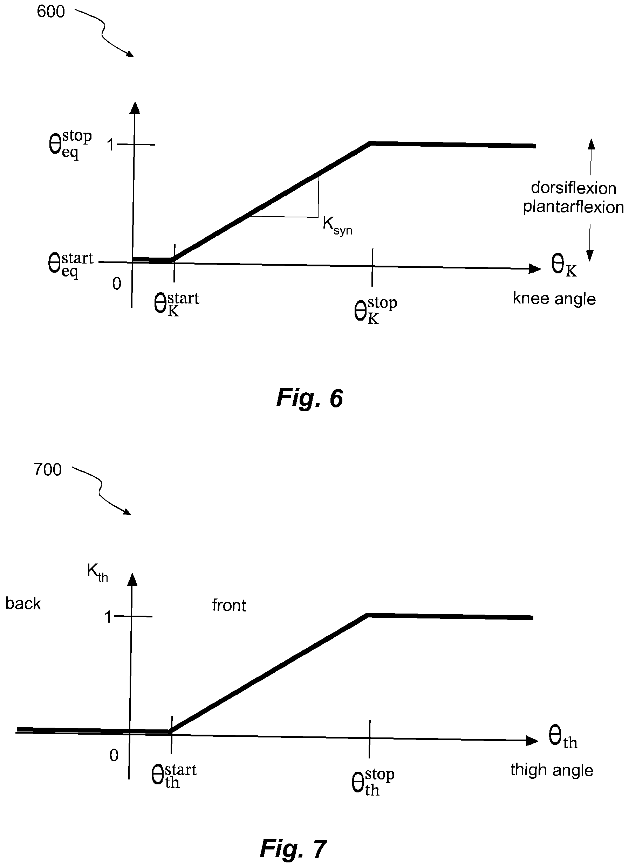

FIG. 6 illustrates the synergistic gain value K_{syn} on a plot 600 of equilibrium angles θ_{eq} versus knee angles θ_{K}. The synergistic gain value K_{syn} can be a slope of a line between two knee angle thresholds, ${θ}_{K}^{start}$ and ${θ}_{K}^{stop}$*,* and two equilibrium angle thresholds, ${θ}_{eq}^{start}$ and ${θ}_{eq}^{stop}$*,* that can be tuned. The knee angle thresholds, ${θ}_{K}^{start}$ and ${θ}_{K}^{stop}$*,* are different from the values for the variable α from the first equilibrium angle θ_{eq1}, discussed above. When the knee is straight during stance, the synergistic gain value K_{syn} can set the second equilibrium angle θ_{eq2} equal to 0. The magnitude of the synergistic gain value K_{syn} can increase as the knee bends faster during swing, and can reach the thresholds faster.

FIG. 7 illustrates a plot 700 of example values of the gain value Kₜₕ versus the thigh angle θₜₕ. The gain value Kₜₕ can increase from 0 to 1 as the thigh moves in front of the body during swing between two thigh angle thresholds, ${θ}_{th}^{start}$ and ${θ}_{th}^{stop}$*.* These thigh angle thresholds, ${θ}_{th}^{start}$ and ${θ}_{th}^{stop}$*,* are different from the values for the variable β from the first equilibrium angle θ_{eq1}, discussed above. When the thigh is behind the body, the gain value Kₜₕ can set the second equilibrium angle θ_{eq2} equal to 0. As the foot leaves the ground during swing, the thigh crosses in front of the body, and the gain value Kₜₕ can increase to 1, turning the second equilibrium angle θ_{eq2} half of the desired equilibrium angle equation on. The first equilibrium angle θ_{eq1} half of the desired equilibrium angle equation is off at this point; so the second equilibrium angle θ_{eq2} half of the desired equilibrium angle equation can provide dorsiflexion assistance to improve foot clearance during swing.

In one aspect, the second equilibrium angle θ_{eq2} can equal a product of a knee angle gain K_{syn} and thigh angle gain Kₜₕ. The knee angle gain K_{syn} can be a slope of a line between two knee angle thresholds and two equilibrium angle thresholds, and can have a value of 0 when the knee angle θ_{K} is straight associated with stance and increases as the knee angle θ_{K} bends faster during swing. The thigh angle gain Kₜₕ can have a value increasing from 0 to 1 as the thigh angle θₜₕ changes with the thigh in front of the body and has a value of 0 when the thigh is behind the body.

Thus, the equilibrium angle *θ_{eq}* can set the assistive torque provided by the controller. The desired equilibrium angle *θ_{eq}* can be the sum of two equilibrium angles *θ*_{*eq*1} and *θ*_{*eq*2}*.* The first equilibrium angle *θ*_{*eq*1} can contribute to dorsiflexion or plantarflexion assistance during stance based on the difference between the ankle and shank angles, θ_{A} and θₛₕ. The first equilibrium angle *θ*_{*eq*1} can contribute to dorsiflexion assistance during swing based on how quickly the knee is bending.

The stiffness of the ankle exoskeleton can increase when the ankle angle θ_{A} is farther from the desired equilibrium angle θ_{eq}. Damping can contribute to the assistive torque based on the ankle velocity and whether the ankle is plantarflexing or dorsiflexing.

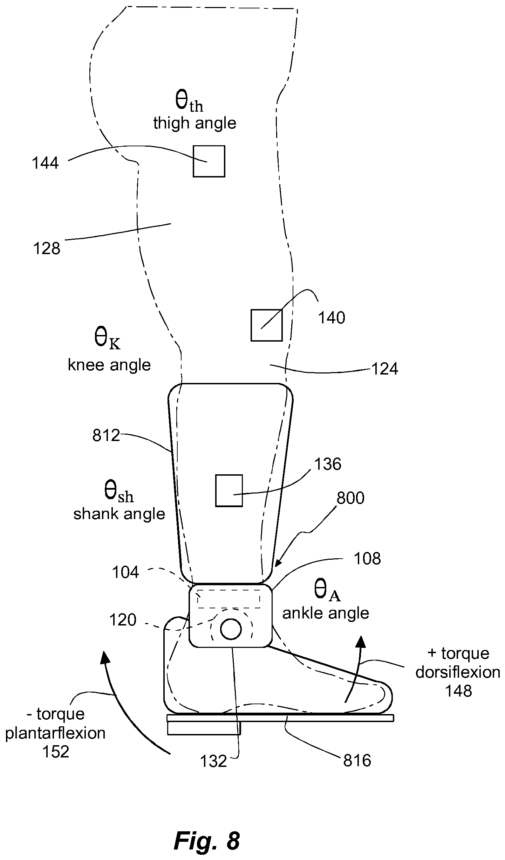

In another example, FIG. 8 illustrates a powered ankle assistive device, namely an ankle exoskeleton 800, with a unified controller 104 in another example of the invention. The ankle exoskeleton 800 and the unified controller 104 are similar in many respects to the ankle prosthesis 100 and the unified controller 104 described above, and which description is incorporated herein by reference. The ankle exoskeleton 800 can comprise an ankle joint assembly 108 configured to be coupled between an upper support member, such as a calf support member 812, and a lower support member, such as a foot support member 816. The ankle exoskeleton 800 can be powered and/or active. Thus, the ankle exoskeleton 800 can have an actuator 120 associated with the ankle joint assembly 108 and configured to move the foot support member 816 with respect to the calf support member 812. The actuator 120 can comprise one or more electric, hydraulic and/or pneumatic linear and/or rotary motors coupled to and between the ankle joint assembly 108 and the foot support member 816.

The controller can comprise one or more digital processors coupled to a memory. The one or more digital processors can be general purpose processors, specialized processors such as very large scale integrated (VLSI) circuits, field programmable gate array processors (FPGAs), or other types of specialized processors, as well as baseband processors used in transceivers to send, receive, and process wireless communications. The controller can comprise a single chip, or multiple chips in a single package, that provides the different processing and transceiver operations described herein. The terms "controller," "processor," "processors," and "one or more processors" are used interchangeably herein.

Various techniques, or certain aspects or portions thereof, can take the form of program code (i.e., instructions) embodied in tangible media, such as floppy diskettes, compact disc-read-only memory (CD-ROMs), hard drives, non-transitory computer readable storage medium, or any other machine-readable storage medium wherein, when the program code is loaded into and executed by a machine, such as a computer, the machine becomes an apparatus for practicing the various techniques. Circuitry can include hardware, firmware, program code, executable code, computer instructions, and/or software. A non-transitory computer readable storage medium can be a computer readable storage medium that does not include a signal. In the case of program code execution on programmable computers, the computing device can include a processor, a storage medium readable by the processor (including volatile and non-volatile memory and/or storage elements), at least one input device, and at least one output device. The volatile and non-volatile memory and/or storage elements can be a random-access memory (RAM), erasable programmable read only memory (EPROM), flash drive, optical drive, magnetic hard drive, solid state drive, or other medium for storing electronic data. One or more programs that can implement or utilize the various techniques described herein can use an application programming interface (API), reusable controls, and the like. Such programs can be implemented in a high level procedural or object oriented programming language to communicate with a computer system. However, the program(s) can be implemented in assembly or machine language, if desired. In any case, the language can be a compiled or interpreted language, and combined with hardware implementations.

As used herein, the term processor can include general purpose processors, specialized processors such as VLSI, FPGAs, or other types of specialized processors, as well as baseband processors used in transceivers to send, receive, and process wireless communications.

It should be understood that many of the functional units described in this specification have been labeled as modules, in order to more particularly emphasize their implementation independence. For example, a module can be implemented as a hardware circuit comprising custom very-large-scale integration (VLSI) circuits or gate arrays, off-the-shelf semiconductors such as logic chips, transistors, or other discrete components. A module can also be implemented in programmable hardware devices such as field programmable gate arrays, programmable array logic, programmable logic devices or the like.

In one example, multiple hardware circuits or multiple processors can be used to implement the functional units described in this specification. For example, a first hardware circuit or a first processor can be used to perform processing operations and a second hardware circuit or a second processor (e.g., a transceiver or a baseband processor) can be used to communicate with other entities. The first hardware circuit and the second hardware circuit can be incorporated into a single hardware circuit, or alternatively, the first hardware circuit and the second hardware circuit can be separate hardware circuits.

Modules can also be implemented in software for execution by various types of processors. An identified module of executable code can, for instance, comprise one or more physical or logical blocks of computer instructions, which can, for instance, be organized as an object, procedure, or function. Nevertheless, the executables of an identified module need not be physically located together, but can comprise disparate instructions stored in different locations which, when joined logically together, comprise the module and achieve the stated purpose for the module.

Indeed, a module of executable code can be a single instruction, or many instructions, and can even be distributed over several different code segments, among different programs, and across several memory devices. Similarly, operational data can be identified and illustrated herein within modules and can be embodied in any suitable form and organized within any suitable type of data structure. The operational data can be collected as a single data set, or can be distributed over different locations including over different storage devices, and can exist, at least partially, merely as electronic signals on a system or network. The modules can be passive or active, including agents operable to perform desired functions.

The technology described here may also be stored on a computer readable storage medium that includes volatile and non-volatile, removable, and non-removable media implemented with any technology for the storage of information such as computer readable instructions, data structures, program modules, or other data. Computer readable storage media include, but is not limited to, a non-transitory machine readable storage medium, such as RAM, ROM, EEPROM, flash memory or other memory technology, CD-ROM, digital versatile disks (DVD) or other optical storage, magnetic cassettes, magnetic tapes, magnetic disk storage or other magnetic storage devices, or any other computer storage medium which may be used to store the desired information and described technology.

The devices described herein may also contain communication connections or networking apparatus and networking connections that allow the devices to communicate with other devices. Communication connections are an example of communication media. Communication media typically embodies computer readable instructions, data structures, program modules, and other data in a modulated data signal such as a carrier wave or other transport mechanism and includes any information delivery media. A "modulated data signal" means a signal that has one or more of its characteristics set or changed in such a manner as to encode information in the signal. By way of example and not limitation, communication media includes wired media such as a wired network or direct-wired connection and wireless media such as acoustic, radio frequency, infrared, and other wireless media. The term computer readable media, as used herein, includes communication media.

Reference was made to the examples illustrated in the drawings and specific language was used herein to describe the same. It will nevertheless be understood that no limitation of the scope of the technology is thereby intended. Alterations and further modifications of the features illustrated herein and additional applications of the examples as illustrated herein are to be considered within the scope of the description.

Furthermore, the described features, structures, or characteristics may be combined in any suitable manner in one or more examples. In the preceding description, numerous specific details were provided, such as examples of various configurations to provide a thorough understanding of examples of the described technology. It will be recognized, however, that the technology may be practiced without one or more of the specific details, or with other methods, components, devices, etc. In other instances, well-known structures or operations are not shown or described in detail to avoid obscuring aspects of the technology.

One, non-limiting embodiment of the present invention will now be described by way of reference to the following clauses:
1. A powered ankle assistive device, comprising:
   an ankle joint assembly configured to be coupled between an upper support member and a lower support member;
   an actuator associated with the ankle joint assembly and configured to move the lower support member with respect to the upper support member;
   one or more angle sensors configured to sense an ankle angle of the ankle joint assembly, a shank angle of the upper support member, a knee angle of a knee associated with the ankle joint assembly, and a thigh angle of a thigh associated with the ankle joint assembly; and
   a controller associated with the ankle joint assembly and configured to:
      receive angle data from the one or more angle sensors comprising the ankle angle, the shank angle, the knee angle and the thigh angle;
      determine a torque to be applied by the actuator based on the angle data; and
      generate a control signal to the actuator to enable the actuator to apply the torque between the ankle joint assembly and the lower support member.
2. The powered ankle assistive device of clause 1, wherein the powered ankle assistive device is configured as an ankle exoskeleton, and wherein:
   the upper support member comprises a calf support member and the lower support member comprises a foot support member; and
   the controller is configured to generate the control signal to the actuator to enable the actuator to apply the torque between the ankle joint assembly and the foot support member.
3. The powered ankle assistive device of clause 1, wherein the powered ankle assistive device is configured as an ankle prosthesis, and wherein:
   the upper support member comprises a shank and the lower support member comprises a prosthetic foot member; and
   the controller is configured to generate the control signal to the actuator to enable the actuator to apply the torque between the ankle joint assembly and the prosthetic foot member.
4. The powered ankle assistive device of any one of clauses 1-3, wherein the controller is further configured to:
   determine a positive dorsiflexion torque based on the angle data associated with a swing and the control signal enables the actuator to apply the positive dorsiflexion torque; and
   determine a positive dorsiflexion torque or a negative plantarflexion torque based on the angle data associated with a stance or a propulsive phase of gait and the control signal enables the actuator to apply the positive dorsiflexion torque or the negative plantarflexion torque.
5. The powered ankle assistive device of any one of clauses 1-4, wherein the controller is further configured to:
   determine a magnitude and a timing of the torque applied by the actuator based on only the angle data.
6. The powered ankle assistive device of any one of clauses 1-5, wherein the controller is further configured to:
   generate the control signal to enable the actuator to continuously apply torque.
7. The powered ankle assistive device of any one of clauses 1-6, wherein the controller is further configured to:
   determine the torque to be applied by the actuator based on:
   a stiffness coefficient applied to a difference between the ankle angle and an ankle equilibrium angle; and
   a damping coefficient applied to an ankle velocity.
8. The powered ankle assistive device of clause 7, wherein at least one of:
   a. the stiffness coefficient decreases as the ankle angle approaches the ankle equilibrium angle;
   b. the stiffness coefficient comprises at least two different stiffness coefficients corresponding to different ranges of the ankle angle associated with different stiffness values, and comprising a first stiffness coefficient corresponding to a first range of ankle angles and a second stiffness coefficient corresponding to a second range of ankle angles; and wherein the second stiffness coefficient is greater than the first stiffness coefficient, and the ankle angles in the second range of ankle angles is greater than the ankle angles in the first range of ankle angles; and
   c. the stiffness coefficient for each of plantarflexion and dorsiflexion comprises at least two different stiffness coefficients corresponding to different ranges of the ankle angle.
9. The powered ankle assistive device of clause 7, wherein at least one of:
   a. the damping coefficient comprises a dorsiflexion damping coefficient corresponding to a positive velocity of the ankle associated with dorsiflexing, and a different plantarflexion damping coefficient corresponding to a negative velocity of the ankle associated with plantarflexing;
   b. the equilibrium angle is based on the knee angle and the thigh angle; and
   c. the equilibrium angle is comprised of two equilibrium angles comprising:
      a first equilibrium angle corresponding to application of positive dorsiflexion torque and negative plantarflexion torque and associated with an early-to-mid stance of gait and a mid-to-late stance, respectively; and
      a second equilibrium angle corresponding to application of positive dorsiflexion torque and associated with a swing.
10. The powered ankle assistive device of clause 9, wherein:
   the first equilibrium angle equals a threshold gain value multiplied by a difference between the ankle angle and the shank angle; and
   the threshold gain value equals 1 less a product of knee angle factor increasing from 0 to 1 as the knee angle increases between two threshold values and thigh angle factor increasing from 0 to 1 as the thigh angle increases between two threshold values.
11. The powered ankle assistive device of clause 9, wherein:
   the second equilibrium angle equals a product of a knee angle gain and thigh angle gain;
   the knee angle gain is a slope of a line between two knee angle thresholds and two equilibrium angle thresholds, and has a value of 0 when the knee angle is straight associated with stance during gait and increases as the knee angle bends faster during swing during gait; and
   the thigh angle gain has a value increasing from 0 to 1 as the thigh angle changes with the thigh in front of a body and has a value of 0 when the thigh is behind the body.
12. The powered ankle assistive device of any one of clauses 1 to 11, wherein the controller is further configured to:
   determine the torque applied by the actuator based on: ${T}_{A} = {K}_{S} \left({θ}_{A}-{θ}_{eq}\right) - B {\dot{θ}}_{A} ;$ where:
   T_{A} is the torque applied;
   K_{S} is a stiffness coefficient;
   θ_{A} is the ankle angle;
   θ_{eq} is an ankle equilibrium angle;
   B is a damping coefficient; and
   *θ̇*_{A} is an ankle velocity.
13. The powered ankle assistive device of clause 12, wherein the equilibrium angle is based on: ${θ}_{eq} = {θ}_{eq 1} + {θ}_{eq 2} ;$ where:
   θ_{eq} is the equilibrium angle;
   θ_{eq1} is a first equilibrium angle corresponding to application of positive dorsiflexion torque and negative plantarflexion torque and associated with an early-to-mid stance and a mid-to-late stance during gait, respective; and
   θ_{eq2} is a second equilibrium angle corresponding to application of positive dorsiflexion torque and associated with a swing during gait.
14. The powered ankle assistive device of clause 13, wherein the first equilibrium angle is based on: ${θ}_{eq 1} = {K}_{ths 1} \left({θ}_{A}-{θ}_{sh}\right) ;$ where:
   Kₜₕₛ₁ is a threshold gain value;
   θ_{A} is the ankle angle; and
   θₛₕ is the shank angle.
15. The powered ankle assistive device of clause 14, wherein the threshold gain value is based on: ${K}_{ths 1} = 1 - αβ ;$ where:
   α has a value increasing from 0 to 1 as the knee angle increases between two threshold values; and
   β has a value increasing from 0 to 1 as the thigh angle increases between two threshold values.
16. The powered ankle assistive device of clause 13, wherein the second equilibrium angle is based on: ${θ}_{eq 2} = {K}_{syn} {K}_{th} ;$ where:
   K_{syn} is a slope of a line between two knee angle thresholds and two equilibrium angle thresholds, and where K_{syn} has a value of 0 when the knee angle is straight associated with stance during gait and where K_{syn} increases as the knee angle bends faster during swing during gait; and
   Kₜₕ has a value increasing from 0 to 1 as the thigh angle changes with the thigh in front of a body and has a value of 0 when the thigh is behind the body.

Although the subject matter has been described in language specific to structural features and/or operations, it is to be understood that the subject matter defined in the appended claims is not necessarily limited to the specific features and operations described above. Rather, the specific features and acts described above are disclosed as example forms of implementing the claims. Numerous modifications and alternative arrangements may be devised without departing from the spirit and scope of the described technology.

## Claims

1. A powered ankle assistive device, comprising:
an ankle joint assembly configured to be coupled between an upper support member and a lower support member;
an actuator associated with the ankle joint assembly and configured to move the lower support member with respect to the upper support member;
one or more angle sensors configured to sense an ankle angle of the ankle joint assembly, a shank angle of the upper support member, a knee angle of a knee associated with the ankle joint assembly, and a thigh angle of a thigh associated with the ankle joint assembly; and
a controller associated with the ankle joint assembly and configured to:
receive angle data from the one or more angle sensors comprising the ankle angle, the shank angle, the knee angle and the thigh angle;
determine a torque to be applied by the actuator based on the angle data; and
generate a control signal to the actuator to enable the actuator to apply the torque between the ankle joint assembly and the lower support member.

2. The powered ankle assistive device of claim 1, wherein the powered ankle assistive device is configured as an ankle exoskeleton, and wherein:
the upper support member comprises a calf support member and the lower support member comprises a foot support member; and
the controller is configured to generate the control signal to the actuator to enable the actuator to apply the torque between the ankle joint assembly and the foot support member.

3. The powered ankle assistive device of claim 1, wherein the powered ankle assistive device is configured as an ankle prosthesis, and wherein:
the upper support member comprises a shank and the lower support member comprises a prosthetic foot member; and
the controller is configured to generate the control signal to the actuator to enable the actuator to apply the torque between the ankle joint assembly and the prosthetic foot member.

4. The powered ankle assistive device of any one of claims 1-3, wherein the controller is further configured to:
determine a positive dorsiflexion torque based on the angle data associated with a swing and the control signal enables the actuator to apply the positive dorsiflexion torque; and
determine a positive dorsiflexion torque or a negative plantarflexion torque based on the angle data associated with a stance or a propulsive phase of gait and the control signal enables the actuator to apply the positive dorsiflexion torque or the negative plantarflexion torque.

5. The powered ankle assistive device of any one of claims 1-4, wherein the controller is further configured to:
determine a magnitude and a timing of the torque applied by the actuator based on only the angle data.

6. The powered ankle assistive device of any one of claims 1-5, wherein the controller is further configured to:
generate the control signal to enable the actuator to continuously apply torque.

7. The powered ankle assistive device of any one of claims 1-3, wherein the controller is further configured to:
determine the torque to be applied by the actuator based on:
a stiffness coefficient applied to a difference between the ankle angle and an ankle equilibrium angle; and
a damping coefficient applied to an ankle velocity.

8. The powered ankle assistive device of claim 7, wherein at least one of:
a. the stiffness coefficient decreases as the ankle angle approaches the ankle equilibrium angle;
b. the stiffness coefficient comprises at least two different stiffness coefficients corresponding to different ranges of the ankle angle associated with different stiffness values, and comprising a first stiffness coefficient corresponding to a first range of ankle angles and a second stiffness coefficient corresponding to a second range of ankle angles; and wherein the second stiffness coefficient is greater than the first stiffness coefficient, and the ankle angles in the second range of ankle angles is greater than the ankle angles in the first range of ankle angles; and
c. the stiffness coefficient for each of plantarflexion and dorsiflexion comprises at least two different stiffness coefficients corresponding to different ranges of the ankle angle.

9. The powered ankle assistive device of claim 7, wherein at least one of:
a. the damping coefficient comprises a dorsiflexion damping coefficient corresponding to a positive velocity of the ankle associated with dorsiflexing, and a different plantarflexion damping coefficient corresponding to a negative velocity of the ankle associated with plantarflexing;
b. the equilibrium angle is based on the knee angle and the thigh angle; and
c. the equilibrium angle is comprised of two equilibrium angles comprising:
a first equilibrium angle corresponding to application of positive dorsiflexion torque and negative plantarflexion torque and associated with an early-to-mid stance of gait and a mid-to-late stance, respectively; and
a second equilibrium angle corresponding to application of positive dorsiflexion torque and associated with a swing.

10. The powered ankle assistive device of claim 9, wherein:
the first equilibrium angle equals a threshold gain value multiplied by a difference between the ankle angle and the shank angle; and
the threshold gain value equals 1 less a product of knee angle factor increasing from 0 to 1 as the knee angle increases between two threshold values and thigh angle factor increasing from 0 to 1 as the thigh angle increases between two threshold values.

11. The powered ankle assistive device of claim 9, wherein:
the second equilibrium angle equals a product of a knee angle gain and thigh angle gain;
the knee angle gain is a slope of a line between two knee angle thresholds and two equilibrium angle thresholds, and has a value of 0 when the knee angle is straight associated with stance during gait and increases as the knee angle bends faster during swing during gait; and
the thigh angle gain has a value increasing from 0 to 1 as the thigh angle changes with the thigh in front of a body and has a value of 0 when the thigh is behind the body.

12. The powered ankle assistive device of claim 1, wherein the controller is further configured to:
determine the torque applied by the actuator based on: ${T}_{A} = {K}_{S} \left({θ}_{A}-{θ}_{eq}\right) - B {\dot{θ}}_{A} ;$ where:
T_{A} is the torque applied;
K_{S} is a stiffness coefficient;
θ_{A} is the ankle angle;
θ_{eq} is an ankle equilibrium angle;
B is a damping coefficient; and
*θ̇*_{A} is an ankle velocity.

13. The powered ankle assistive device of claim 12, wherein the equilibrium angle is based on: ${θ}_{eq} = {θ}_{eq 1} + {θ}_{eq 2} ;$ where:
θ_{eq} is the equilibrium angle;
θ_{eq1} is a first equilibrium angle corresponding to application of positive dorsiflexion torque and negative plantarflexion torque and associated with an early-to-mid stance and a mid-to-late stance during gait, respective; and
θ_{eq2} is a second equilibrium angle corresponding to application of positive dorsiflexion torque and associated with a swing during gait.

14. The powered ankle assistive device of claim 13, wherein the first equilibrium angle is based on: ${θ}_{eq 1} = {K}_{ths 1} \left({θ}_{A}-{θ}_{sh}\right) ;$ where:
Kₜₕₛ₁ is a threshold gain value;
θ_{A} is the ankle angle; and
θₛₕ is the shank angle, wherein preferably the threshold gain value is based on: ${K}_{ths 1} = 1 - αβ ;$
where:
α has a value increasing from 0 to 1 as the knee angle increases between two threshold values; and
β has a value increasing from 0 to 1 as the thigh angle increases between two threshold values.

15. The powered ankle assistive device of claim 13, wherein the second equilibrium angle is based on: ${θ}_{eq 2} = {K}_{syn} {K}_{th} ;$ where:
K_{syn} is a slope of a line between two knee angle thresholds and two equilibrium angle thresholds, and where K_{syn} has a value of 0 when the knee angle is straight associated with stance during gait and where K_{syn} increases as the knee angle bends faster during swing during gait; and
Kₜₕ has a value increasing from 0 to 1 as the thigh angle changes with the thigh in front of a body and has a value of 0 when the thigh is behind the body.
